# EUROPÄISCHE PATENTSCHRIFT

(11) **EP 3 342 436 B1**
(45) Veröffentlichungstag und Bekanntmachung des Hinweises auf die Patenterteilung: **15.01.2020**
(21) Anmeldenummer: 17002042.4
(22) Anmeldetag: 20.12.2017
(51) Int. Cl.: A61M 5/178, A61J 1/00, A61B 5/145, A61B 5/153, G01N 1/18, B01L 3/02, G01N 33/49, B01L 3/00, A61B 5/15, A61M 1/02

(54) **VORRICHTUNG ZUM AUFNEHMEN VON FLÜSSIGKEITEN SOWIE ZUR EXAKTEN ENTNAHME VON EINZELNEN PHASEN DER AUFGENOMMENEN FLÜSSIGKEIT**
DEVICE FOR RECEIVING LIQUIDS AND FOR EXACTLY REMOVING SINGLE PHASES OF THE RECEIVED LIQUID
DISPOSITIF POUR RECEVOIR DES LIQUIDES ET POUR PRÉLEVER EXACTEMENT DES PHASES UNIQUES DU LIQUIDE RECUE.

(30) Priorität: 29.12.2016 DE 202016008016 U
(43) Veröffentlichungstag der Anmeldung: 04.07.2018
(73) Patentinhaber: Rohrer, Heinz, 83022 Rosenheim (DE)
(72) Erfinder: Rohrer, Heinz, 83022 Rosenheim (DE)

(56) Entgegenhaltungen:
- EP-A1- 2 077 115
- WO-A2-2011/019366
- US-A- 4 660 569
- US-A1- 2014 251 141

## Beschreibung

Die vorliegende Erfindung betrifft eine Vorrichtung zum Aufnehmen von Flüssigkeiten sowie zur exakten Entnahme von einzelnen Phasen der aufgenommenen Flüssigkeit, mit den in dem Patentanspruch 1 angegebenen Merkmalen.

Zum Stand der Technik gehört es, dass man beispielsweise in einem mit einem Stöpsel verschlossenen Reagenzglas venös abgenommenes Blut zentrifugiert, bis es zur Ausbildung a) einer sich im bodennahen Bereich bildenden, voluminösen und dunkelroten Hämatokrit-Phase hoher Dichte, b) einer sich darüber befindlichen, hellen, silbrig schimmernden Grenzschicht-Phase geringen Volumens, geringerer Dichte und mit einem besonders hohen Anteil an Wachstumsfaktoren und c) einer sich darüber befindlichen, voluminösen, meist honiggelben und transparenten Plasma-Phase noch geringerer Dichte, kommt.

Nach der Zentrifugierung wird mit einer Kanüle dort der Stöpsel des Reagenzglases durchstochen und die gewünschte Phase - beispielsweise mittels einer Spritze - entnommen.

Dieses aus dem Stand der Technik bekannte Verfahren zur Gewinnung einzelner Phasen von zentrifugiertem Blut ist unter vielerlei Aspekten nachteilig:
Ein wesentlicher Nachteil dieses bekannten Verfahrens besteht darin, dass es besonders umständlich und zeitaufwändig ist.

Ein weiterer Nachteil dieses bekannten Verfahrens liegt darin, dass es mit der Gefahr der Einbringung von Keimen einhergeht.

Nachteilig ist dieses bekannte Verfahren auch deswegen, weil es die durchmischungsfreie Entnahme von dünnen Grenzschichten kaum erlaubt.

Schließlich ist dieses bekannte Verfahren auch deswegen von Nachteil, weil dort die Ausbeute an entnommenem Volumen der jeweils zu entnehmenden Phase eingeschränkt ist. Schließlich muss dort - zur Verringerung einer Durchmischungsgefahr mit der nachfolgenden Phase - die Entnahme der vorausgehenden Phase deutlich vor ihrer vollständigen Entnahme abgebrochen werden.

Aus der US 4 660 569 A ist eine Spritze zur Entnahme von arteriellem Blut für die Analyse von in dem Blut enthaltenem Gas beschrieben, bei welcher ein gasdurchlässiger, nicht jedoch flüssigkeitsdurchlässiger Pfropfen in dem Kanal eines Zylinderstabes vorgesehen ist. Die Öffnung am rückseitigen, freien Ende des Zylinderstabes wird dort mit einem Stöpsel (35) während des aspiratorischen Ansaugens von Blut verschlossen.

Aufgabe der vorliegenden Erfindung ist daher die Bereitstellung einer Vorrichtung zum Aufnehmen von Flüssigkeiten - insbesondere von Blut - sowie zur exakten Entnahme von durch Sedimentation erzeugten einzelnen Phasen der aufgenommenen Flüssigkeit, welche eine besonders einfache, schnelle und exakte Gewinnung einzelner Phasen gestattet, welche die Gefahr einer Einbringung von Keimen während der Entnahme von einzelnen Phasen sicher ausschließt, welche die Entnahme selbst sehr dünner Grenzschichten - unter Ausschluss der Gefahr von Durchmischung mit der darüber oder darunter befindlichen Phase - gestattet und welche die vollständige Entnahme des Volumens der jeweils interessierenden, einzelnen Phase erlaubt.

Erfindungsgemäß wir diese Aufgabe bei einer gattungsgemäßen Vorrichtung durch die in dem Patentanspruch 1 angegebenen Merkmale gelöst. Besonders bevorzugte Ausführungsformen sind Gegenstände der Unteransprüche.

Ausführungsbeispiele der Erfindung werden anhand der Zeichnungen näher beschrieben. Es zeigen:

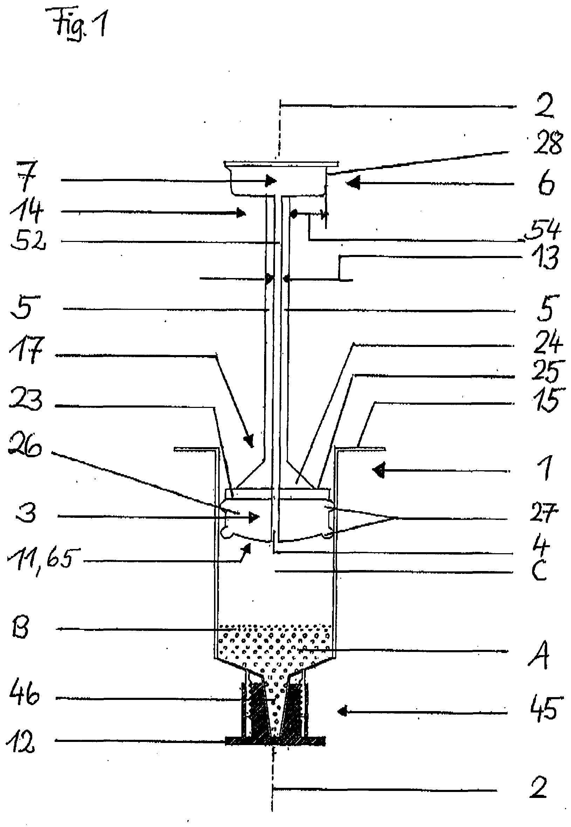

Figur 1 einen schematischen und vertikalen Längsschnitt durch eine erfindungsgemäße Vorrichtung mit einem transparenten Rohr (5), welches stangenseitig an einem Kolben (3) angebracht ist und an seinem freien Ende (14) eine Verschlusskappe (6) trägt, wobei in dem Zylinder (1) - nach der Einwirkung von Sedimentationskräften - drei Phasen A, B und C einer Flüssigkeit ausgebildet sind und sich der Kolben (3) in der Nähe der großen, obenliegenden Öffnung des Zylinders (1) zur Kolbeneinführung, befindet;

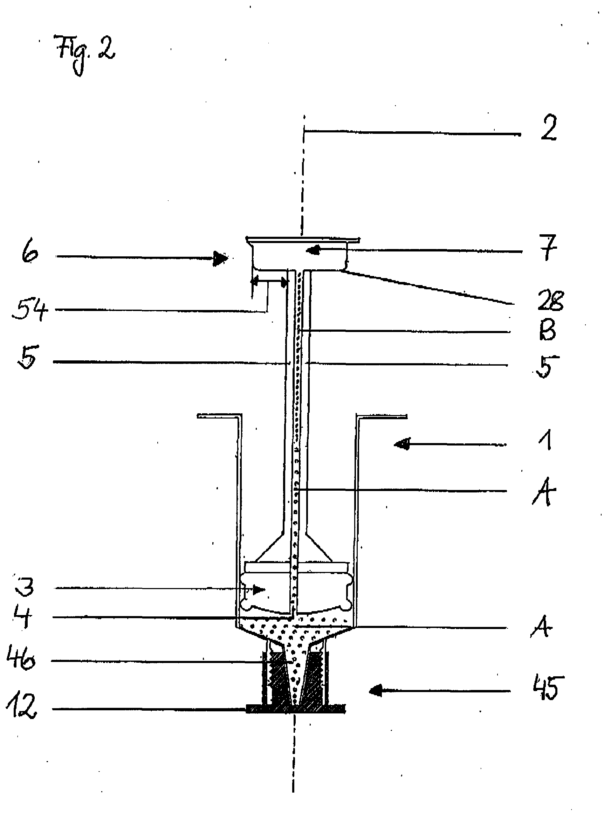

Figur 2 einen schematischen und vertikalen Längsschnitt durch eine erfindungsgemäße Vorrichtung mit einem transparenten Rohr (5), welches stangenseitig an einem Kolben (3) angebracht ist und an seinem freien Ende (14) eine Verschlusskappe (6) trägt, wobei der Kolben (3) in dem Zylinder (1) in Richtung der Spitze (46) des Zylinders (1) nach unten geschoben ist, wobei die Phase A der höchsten Dichte von unten etwa bis zur Mitte des transparenten Rohres (5) - durch die Absenkung des Kolbens (3) - durch die lochförmige Aussparung (4) des Kolbens (3) hindurch, hinauf gedrückt wurde und sich in dem oberhalb der Mitte des transparenten Rohres (5) gelegenen Abschnitt des Rohres (5), noch Flüssigkeit der bereits zum größten Teil aus dem Zylinder (1) entnommenen Phase B befindet;

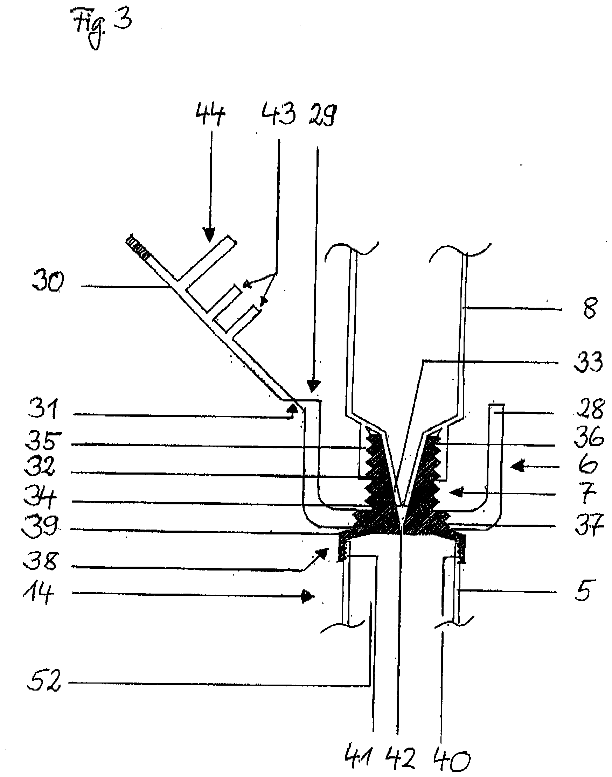

Figur 3 einen schematischen und vertikalen Längsschnitt durch eine in den Figuren 1 und 2 dargestellte Verschlusskappe (6) an dem freien, nach oben ragenden Ende (14) des transparenten Rohres (5), wobei deren Deckel (30) geöffnet ist, um ein dichtes Ansetzen einer Spritze (8) an der Anschlusseinrichtung (7) der Verschlusskappe (6) zu ermöglichen;

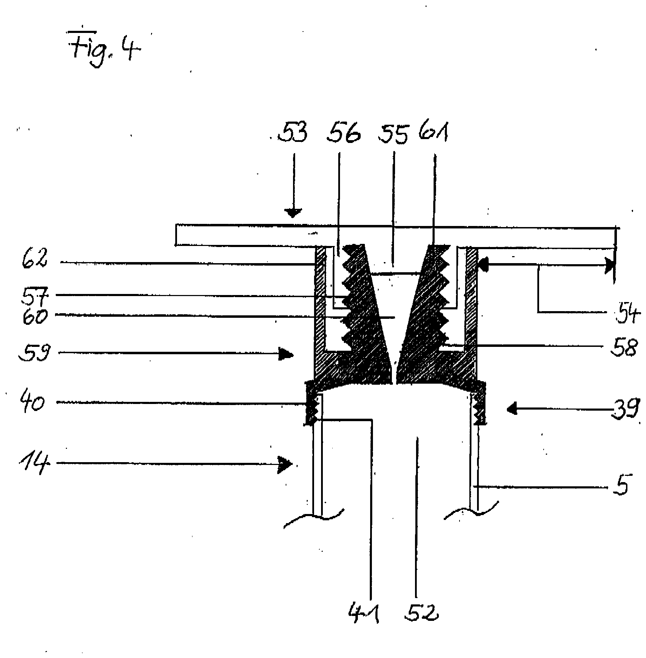

Figur 4 einen schematischen und vertikalen Längsschnitt durch das freie, obere Ende (14) eines erfindungsgemäß verwendeten, transparenten Rohres (5), welches - anstelle einer Verschlusskappe (6) - einen auf eine Anschlusseinrichtung (59) für eine Spritze (8) aufschraubbaren Verschluss-Deckel (53) trägt, wobei der Sockel (39) der Anschlusseinrichtung (59) auf das freie, obere Ende (14) des transparenten Rohres (5) aufschraubbar ist;

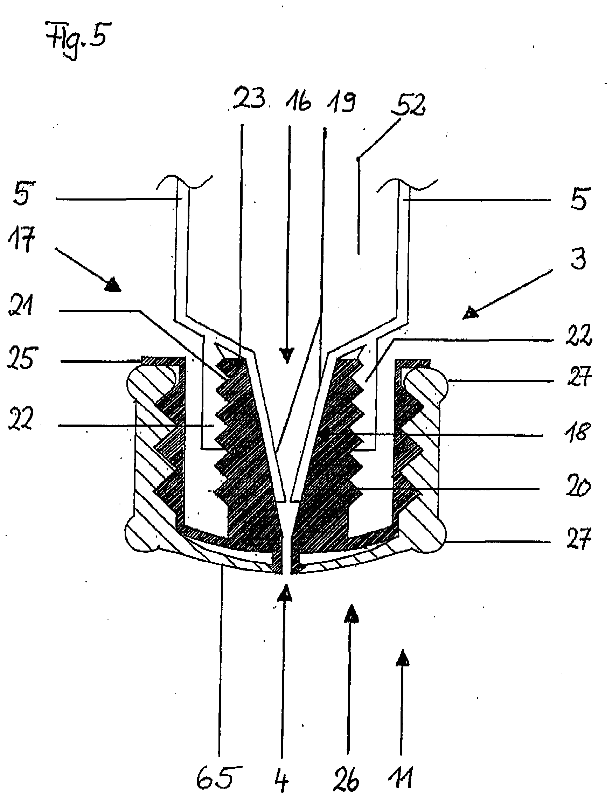

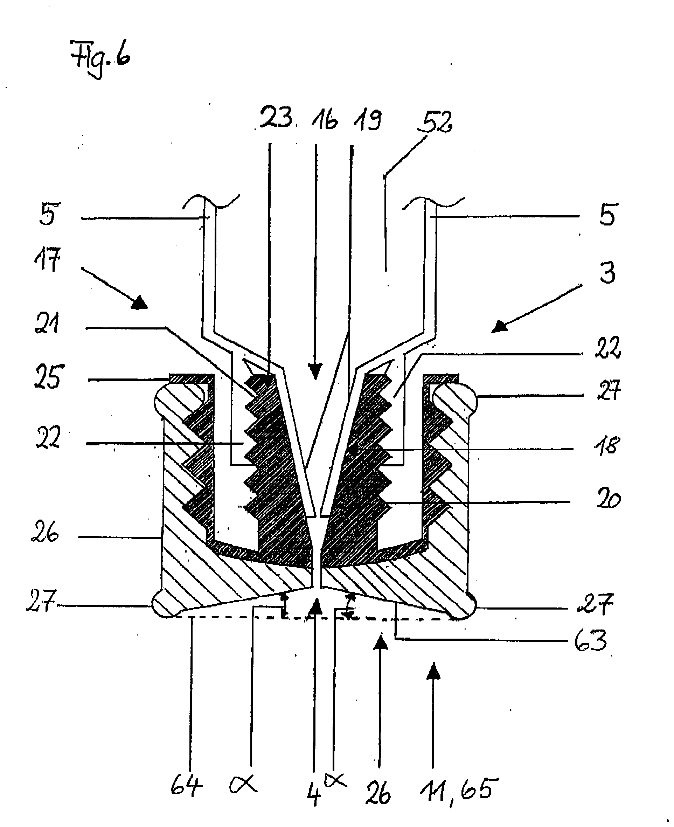

Figur 5 einen schematischen und vertikalen Längsschnitt durch einen in den Figuren 1 und 2 dargestellten Kolben (3), wobei an dem Kolbenträger (23) - im Gegensatz zu dem in den Figuren 1 und 2 dargestellten, einstückig mit dem Kolbenträger (23) ausgebildeten Rohr (5) - das transparente Rohr (5) an einer kolbenseitigen Anschlusseinrichtung (16) mittels eines Konus (18) und eines Außengewindes (20) reversibel anbringbar ist, wobei die bodenseitige Wandung (63) des Bodens (65) der kappenförmigen Dichtung (26) des Kolben-Trägers (23) bodenseitig gewölbt ist, so dass zwischen der nach unten gekrümmten, bodenseitigen Wandung (63) des Bodens (65) der Dichtung (26) einerseits und einer zwischen den unteren ringförmig umlaufenden, gegenüberliegenden Dichtungswülsten (27) der kappenförmigen Dichtung (26) gedachten - aus Übersichtlichkeitsgründen nur in der Figur 6 dargestellten - Horizontale (64) andererseits, ein negativer Winkel α eingeschlossen ist, welcher in dem unterhalb der Horizontalen (64) befindlichen Bereich von 0° bis - 35° liegt;
Figur 6 einen schematischen und vertikalen Längsschnitt durch einen erfindungsgemäßen Kolben (3), wobei an dem Kolbenträger (23) - im Gegensatz zu dem in den Figuren 1 und 2 dargestellten, einstückig mit dem Kolbenträger (23) ausgebildeten Rohr (5) - das transparente Rohr (5) an einer kolbenseitigen Anschlusseinrichtung (16) mittels eines Konus (18) und eines Außengewindes (20) reversibel anbringbar ist und wobei die bodenseitige Wandung (63) des Bodens (65) der kappenförmigen Dichtung (26) des Kolben-Trägers (23) trichterförmig nach oben gewölbt ausgebildet ist, so dass zwischen der nach oben gewölbten bodenseitigen Wandung (63) des Bodens (65) der Dichtung (26) einerseits und einer zwischen den unteren ringförmig umlaufenden, gegenüberliegenden Dichtungswülsten (27) der kappenförmigen Dichtung (26) gedachten Horizontalen (64) andererseits, ein positiver Winkel α eingeschlossen ist, welcher in dem oberhalb der Horizontalen (64) befindlichen Bereich von 0° bis + 35° liegt;

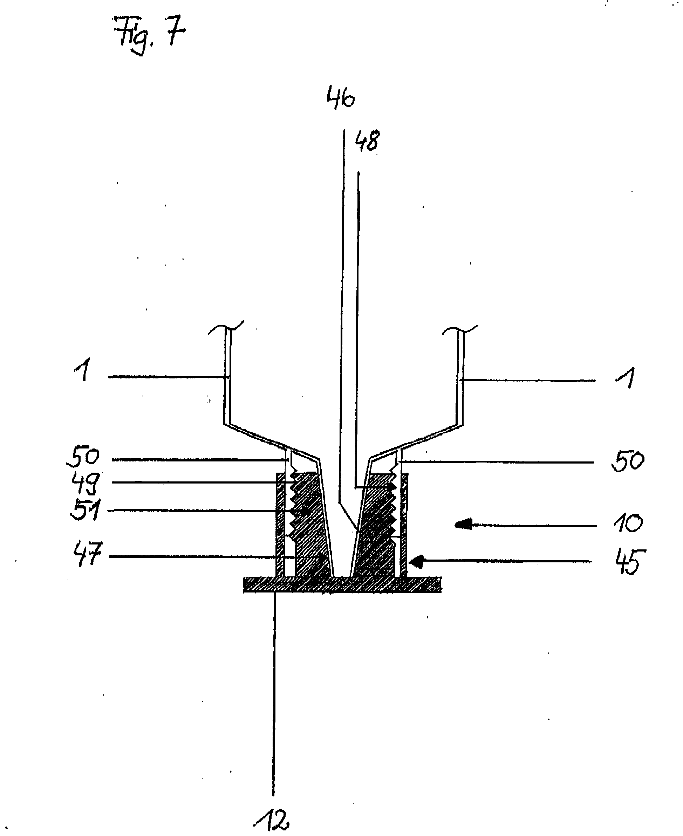

Figur 7 einen schematischen und vertikalen Längsschnitt durch einen in den Figuren 1 und 2 dargestellten Standfuß-Verschluss (45, 12) der Spitze (46) des Zylinders (1), wobei dieser Verschluss (45) einen Konus (47) und ein Außengewinde (48) zum flüssigkeitsdichten Verschließen der Spitze (46) des Zylinders (1) und einen Standfuß (12) - zum vertikal ausgerichteten Aufstellen des Zylinders (1) - umfasst;

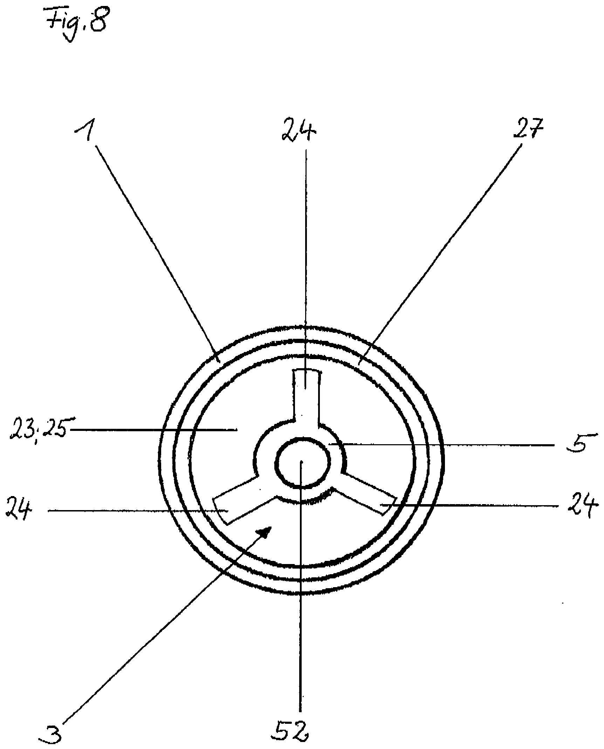

Figur 8 eine schematische, stangenseitige und teilweise horizontal geschnittene Rückansicht auf einen Zylinder (1) mit einem darin vorgesehenen, erfindungsgemäß ausgestalteten Kolben (3), welcher stangenseitig ein zentrales transparentes Rohr (5) trägt, wobei vier jeweils um 90 ° zueinander versetzte Versteifungselemente (24) um das zentrale Rohr (5) herum angeordnet sind, wobei sich die vertikale Länge jedes Versteifungselementes (24) etwa über die gesamte vertikale Länge des zentralen Rohres (5) erstreckt und wobei sich die horizontale Breite jedes Versteifungselementes (24) etwa über die gesamte horizontale Breite des stangenseitigen Bodens (25) des Kolbens (3) zwischen der Außenseite des zentralen Rohres (5) und dem außenliegenden, kreisförmigen Rand des Bodens (25) des Kolbens (3) erstreckt, um durch eine Abstützung an den Innenwänden des Zylinders (1) ein seitliches Kippen des Kolbens (3) während der Einwirkung von Sedimentationskräften zu verhindern;

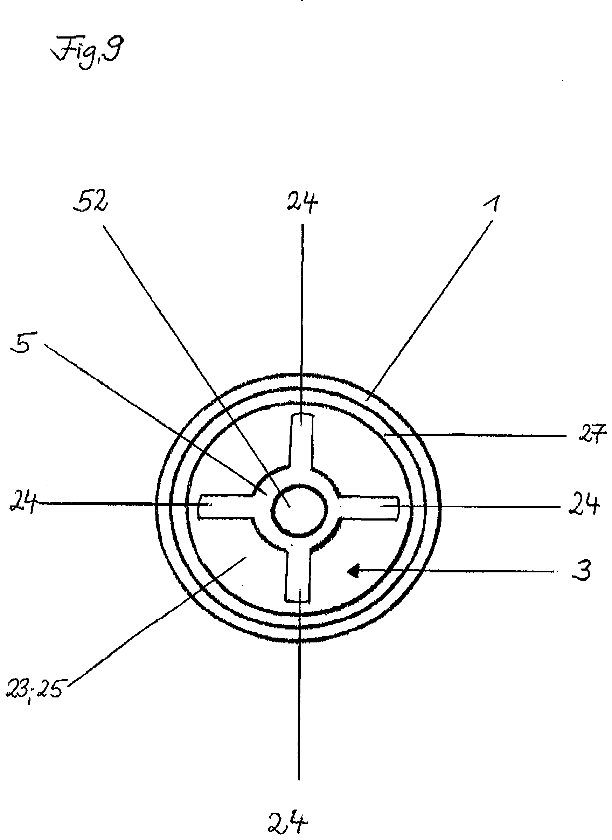

Figur 9 eine schematische, stangenseitige und teilweise horizontal geschnittene Rückansicht auf einen Zylinder (1) mit einem darin vorgesehenen, erfindungsgemäß ausgestalteten Kolben (3), welcher stangenseitig ein zentrales transparentes Rohr (5) trägt, wobei drei jeweils um 120 ° zueinander versetzte Versteifungselemente (24) um das zentrale Rohr (5) herum angeordnet sind, wobei sich die vertikale Länge jedes Versteifungselementes (24) etwa über die gesamte vertikale Länge des zentralen Rohres (5) erstreckt und wobei sich die horizontale Breite jedes Versteifungselementes (24) etwa über die gesamte horizontale Breite des stangenseitigen Bodens (25) des Kolbens (3) zwischen der Außenseite des zentralen Rohres (5) und dem außenliegenden, kreisförmigen Rand des Bodens (25) des Kolbens (3) erstreckt, um durch eine Abstützung an den Innenwänden des Zylinders (1) ein seitliches Kippen des Kolbens (3) während der Einwirkung von Sedimentationskräften zu verhindern.

Wie insbesondere aus den Figuren 1 und 2 hervorgeht, beschreibt die vorliegende Erfindung eine Vorrichtung zum Aufnehmen von Flüssigkeiten sowie zur exakten Entnahme von einzelnen Phasen der aufgenommenen Flüssigkeit.

In der Regel umfasst die erfindungsgemäße Vorrichtung einen Zylinder (1), in welchem ein Kolben (3) entlang der Längsachse (2) des Zylinders (1) reversibel hin- und her- bewegbar ist.

In besonders bevorzugten Ausführungsformen der erfindungsgemäßen Vorrichtung kann der Kolben-Boden (11, 65) - in der Draufsicht - in seinem Mittenbereich eine lochförmige Aussparung (4) aufweisen (siehe insbesondere Figuren 1 und 2 sowie 5 und 6).

In der Regel kann diese lochförmige Aussparung (4) des Kolben-Bodens (11, 65) - durch den Kolben (3) hindurch - mit einem entlang der Längsachse (2) des Zylinders (1) ausgerichteten, sich stangenseitig erstreckenden und vollständig oder teilweise transparenten Rohr (5) mittelbar oder unmittelbar in dichter Verbindung stehen oder mittelbar oder unmittelbar in dichte Verbindung hiermit bringbar sein.

In besonders bevorzugten Ausführungsformen der erfindungsgemäßen Vorrichtung kann der Innendurchmesser (13) des Lumens (52) des vollständig oder teilweise transparenten Rohres (5) in dem Bereich von 0,5 mm bis 10,0 mm, vorzugsweise in dem Bereich von 1,0 mm bis 8,0 mm, insbesondere in dem Bereich von 1,5 mm bis 7,0 mm, liegen.

Im Allgemeinen kann die Länge des vollständig oder teilweise transparenten Rohres (5) - und dessen Lumens (52) - in dem Bereich von 5,0 cm bis 15,0 cm, vorzugsweise in den Bereich von 5,5 cm bis 11,0 cm, insbesondere in dem Bereich von 6,0 cm bis 9,0 cm, liegen.

In besonders bevorzugten Ausführungsformen der erfindungsgemäßen Vorrichtung kann an dem - bei vertikaler Ausrichtung des Zylinders (1) - in vertikaler Hinsicht nach oben ragenden, freien Ende (14) des Rohres (5) eine reversibel verschließbare Verschlusskappe (6) vorgesehen sein (siehe Figur 1, 2 und 3).

Alternativ hierzu kann - statt dieser Verschlusskappe (6) - eine Verschlussschraube oder ein Verschlussdeckel (53) reversibel anbringbar sein (siehe insbesondere Figur 4).

Wie insbesondere aus Figur 3 hervorgeht, kann in der Verschlusskappe (6) eine schraubgewindeförmige und/oder konusförmige Anschlusseinrichtung (7) für eine Injektionsspritze (8) vorgesehen sein.

Der sich über die Außenseite des Rohres (5) horizontal nach außen erstreckende Überstand (54) der Verschlusskappe (6), der Verschlussschraube oder des Verschlussdeckels (53) kann - zur Fingerauflage während des aspiratorischen Anhebens des Kolbens (3) - in dem Bereich von 5,0 mm bis 40,0 mm, vorzugsweise in dem Bereich von 7,0 mm bis 35,0 mm, insbesondere in dem Bereich von 9,0 mm bis 30,0 mm, liegen.

Insbesondere aus den Figuren 1, 2 und 7 ist ersichtlich, dass in dem Bereich der Mitte des Bodens (9) des Zylinders (1) außenseitig eine schraubengewindeförmige und/oder konusförmige Anschlusseinrichtung (10) für eine Kanüle oder für einen aufschraubbaren oder aufsteckbaren Standfuß (12) vorgesehen sein können.

Den Figuren 1 und 2 ist zu entnehmen, dass in dem dem Boden (9) gegenüberliegenden, oberen Bereich des freien Endes des Zylinders (1) außen umlaufend oder gegenüberliegend Vorsprünge (15) zur Fingerauflage vorgesehen sein können.

In der Regel kann das vollständig oder teilweise transparente Rohr (5) - mit seinem Lumen (52) - an der Stangenseite (17) des Kolbens (3) beispielsweise mittels einer permanent fixierenden oder reversibel lösbaren Einrast-, Schraub-, Schnapp-, Steck-, Dreh- oder einer Bajonette-Verbindung anbringbar sein (Schraubverbindung siehe beispielsweise Figuren 5 und 6).

Insbesondere aus den Figuren 5 und 6 geht hervor, dass das vollständig oder teilweise transparente Rohr (5) - mit seinem Lumen (52) - an der Stangenseite (17) des Kolbens (3) mittels einer konusförmigen und/oder schraubgewindeförmigen, vorzugsweise in den Kolben (3) integrierten Anschlusseinrichtung (16) reversibel anbringbar sein kann.

Wie in den Figuren 5 und 6 gezeigt, kann die Anschlusseinrichtung (16) beispielsweise einen zentralen Konus (18) für die Ausrichtung und Arretierung einer Spitze (19) des Rohres (5) umfassen.

Vorzugsweise kann außenseitig an einem ringförmig den Konus (18) wandartig umschließenden Element (22) ein Außengewinde (20) für das Aufschrauben des Innengewindes (21) eines ringförmig die Spitze (19) des Rohres (5) umschließenden Elementes (22) vorgesehen sein.

Wie insbesondere in den Figuren 1 und 2 dargestellt, kann das vollständig oder teilweise transparente Rohr (5) - mit seinem Lumen (52) - einstückig mit der Stangenseite (17) des Kolbenträgers (23) des Kolbens (3) ausgebildet sein.

Vorzugsweise können dann zwischen den Außenseiten des Rohres (5) und dem stangenseitigen Boden (25) des Kolbens (3) ein, zwei, drei, vier, fünf, sechs oder mehrere Versteifungselemente (24) vorgesehen sein.

Insbesondere in den Figuren 8 und 9 ist dargestellt, dass sich die Versteifungselemente (24) in vertikaler Hinsicht über die gesamte vertikale Höhe des Rohres (5) - oder über einen Teil hiervon (Figuren 1 und 2) - erstrecken können.

Ebenfalls aus den Figuren 1, 2 und 8, 9 ergibt sich, dass sich die Versteifungselemente (24) in horizontaler Hinsicht, beispielsweise über die gesamte Breite des stangenseitigen Bodens (25) des Kolbenträgers (23) - oder über einen Teil hiervon - zwischen der Außenseite des zentralen Rohres (5) und dem außenliegenden, kreisförmigen Rand des Bodens (25) des Kolbens (3), erstrecken können.

Die gegebenenfalls vorgesehenen Versteifungselemente (24) führen einerseits zu dem Vorteil, dass das zentrale, vollständig oder teilweise transparente Rohr (5) stabil mit dem stangenseitigen Boden (25) des Kolbens (3) in Verbindung steht. Andererseits führen diese Versteifungselemente (24) zu dem Vorteil, dass durch eine Abstützung der in vertikaler Hinsicht hochgezogenen Versteifungselemente (24) an den Innenwänden des Zylinders (1) ein seitliches Kippen des Kolbens (3) - beispielsweise während der Einwirkung von Sedimentationskräften - sicher verhindert werden kann.

In besonders bevorzugten Ausführungsformen der erfindungsgemäßen Vorrichtung kann der Kolbenträger (23) - zur Abdichtung des Kolbens (3) gegenüber den Innenwänden des Zylinders (1) - beispielsweise eine kappenförmige Dichtung (26) mit einem, zwei, drei, vier, fünf oder sechs Dichtungswülsten (27) tragen (siehe Figuren 1, 2, 5 und 6).

In der Regel kann die Dichtung (26) vorzugsweise aus einem elastischen Material gefertigt sein.

Vorzugsweise kann in dem Boden (65) der kappenförmigen Dichtung (26) eine zentrale lochförmige Aussparung vorgesehen sein, welche mit der lochförmigen Aussparung (4) des Kolbens (3) und mit dem Lumen (52) des Rohres (5) durchgehend fluchtet.

Aus den Figuren 1, 2 und 5 geht hervor, dass zwischen der bodenseitigen, gegebenenfalls vertikal nach unten gekrümmten Wandung (63) des Bodens (65) der kappenförmigen Dichtung (26) des Kolben-Trägers (23) einerseits und einer zwischen den unteren ringförmig umlaufenden, gegenüberliegenden Dichtungswülsten (27) der kappenförmigen Dichtung (26) gedachten Horizontalen (64) andererseits, ein Winkel α eingeschlossen sein kann, welcher beispielsweise in dem Bereich von 0° bis -35 °, vorzugsweise in dem Bereich von 0° bis - 30°, insbesondere in dem Bereich von 0° bis - 25°, liegt.

Negative Winkel α (siehe Figuren 1, 2 und 5) betreffen im Rahmen der vorliegenden Erfindung stets nach unten gewölbte Wandungen (63) des Bodens (65) der kappenförmigen Dichtung (26) des Kolben-Trägers (23), wobei die Längsachse des mit dem Kolben (3) in Verbindung stehenden Rohres (5) vertikal ausgerichtet und der Boden (11) des Kolbens (3) nach unten ausgerichtet ist.

Alternativ hierzu kann - wie insbesondere in Figur 6 dargestellt - der Boden (65) der kappenförmigen Dichtung (26) des Kolben-Trägers (23) beispielsweise im Wesentlichen trichterförmig nach oben gewölbt ausgebildet sein.

Der zwischen der trichterförmigen, bodenseitigen Wandung (63) des Bodens (65) der kappenförmigen Dichtung (26) des Kolben-Trägers (23) einerseits und einer zwischen den unteren ringförmig umlaufenden, gegenüberliegenden Dichtungswülsten (27) der kappenförmigen Dichtung (26) gedachten Horizontalen (64) andererseits eingeschlossene Winkel α kann dann beispielsweise in dem Bereich von + 35° bis 0°, vorzugsweise in dem Bereich von + 30° bis 0°, insbesondere in dem Bereich von + 25° bis 0°, liegen.

Positive Winkel α (Figur 6) betreffen im Rahmen der vorliegenden Erfindung stets trichterförmig nach oben gewölbte Wandungen (63) des Bodens (65) der kappenförmigen Dichtung (26) des Kolben-Trägers (23), wobei die Längsachse des mit dem Kolben (3) in Verbindung stehenden Rohres (5) vertikal ausgerichtet und der Boden (11) des Kolbens (3) nach unten ausgerichtet ist.

Die Vorteile der in Figur 6 dargestellten trichterförmigen, nach oben gewölbten Ausgestaltung des Bodens (65) der kappenförmigen Dichtung (26) des Kolben-Trägers (23) - mit einem positiven Winkel α - bestehen insbesondere darin, dass einzelne Phasen mit jeweils unterschiedlichen Dichten besonders scharf und exakt voneinander trennbar sind und die Ausbeuten der jeweils entnommenen Phase besonders groß sind:
Denn selbst in den randseitigen Bereichen der Dichtungswülste (27) befindliche Volumina der zu entnehmenden Phase können dank der trichterförmigen, nach oben gewölbten Ausgestaltung des Bodens (65) der kappenförmigen Dichtung (26) des Kolben-Trägers (23) dann sicher von dem Kolben (3) erfasst und in ihrer Gesamtheit dem Zylinder (1) - unter Ausschluss einer Vermischungsgefahr mit benachbarten Phasen - entnommen werden.

Wie in den Figuren 1, 2 und 3 dargestellt, kann die Verschlusskappe (6) beispielsweise einen im Querschnitt U-förmigen oder einen im Querschnitt einem um 90° zur Seite geneigten Buchstaben C entsprechenden Behälter (28) umfassen.

In besonders bevorzugten Ausführungsformen der erfindungsgemäßen Verschlusskappe (6) kann mittelbar oder unmittelbar an einem Bereich des freien Randes (29) dieses Behälters (28) ein Deckel (30) für das mittelbare oder unmittelbare Verschließen der obenliegenden Öffnung des Behälters (28) vorgesehen sein.

Dieser Deckel (30) kann zusätzlich für das mittelbare oder unmittelbare Verschließen des - innerhalb des Behälters (28) vorgesehenen - Konus (32) einer Anschlusseinrichtung (7) für eine dichte Aufnahme der Spitze (33) einer Spritze (8) dienen.

In besonders bevorzugten Ausführungsformen der erfindungsgemäßen Vorrichtung kann der Deckel (30) beispielsweise über ein Gelenk (31) reversibel und mittelbar oder unmittelbar an dem Behälter (28) schwenkbar angebracht sein.

Insbesondere die Figur 3 zeigt, dass in dem untenliegenden Bodenbereich des Behälters (28) der Verschlusskappe (6) eine vertikal nach oben ausgerichtete Anschlusseinrichtung (7) für eine Spritze (8) vorgesehen sein kann.

Diese Anschlusseinrichtung (7) kann einen Konus (32) zur Zentrierung und Arretierung der Spitze (33) einer Spritze (8) aufweisen.

Zusätzlich hierzu kann die Anschlusseinrichtung (7) ein Außengewinde (34) für den Eingriff eines die Spitze (33) ringförmig umfassenden, an einem ringförmigen Vorsprung (35) angebrachten Innengewindes (36) aufweisen.

Im Allgemeinen können der Behälter (28) und die Anschlusseinrichtung (7) für eine Spritze (8) einstückig miteinander ausgebildet sein.

Alternativ hierzu können der Behälter (28) und die Anschlusseinrichtung (7) über ein Gewinde (37), eine Einrast-, Schnapp-, Steck-, Dreh- oder einer Bajonette- Verbindung miteinander in permanente oder reversible Verbindung bringbar sein.

Wie insbesondere in Figur 3 dargestellt, kann die Anschlusseinrichtung (7) für eine Spritze (8) stromabwärts und in vertikal nach unten ausgerichteter Richtung mittelbar oder unmittelbar mit einer Anschlusseinrichtung (38) für das freie, obere Ende des Rohres (5) in Verbindung stehen.

Figur 3 zeigt ferner, dass an der Innenseite des Deckels (30) eine ein- oder mehrteilige Einrichtung (43) zum mittelbaren oder unmittelbaren, dichten Verschließen des Konus (32) der Anschlusseinrichtung (7) vorgesehen sein kann.

Der Vorteil dieser gegebenenfalls vorhandenen Einrichtung (43) zum mittelbaren oder unmittelbaren, dichten Verschließen des Konus (32) der Anschlusseinrichtung (7) besteht darin, dass selbst bei der Einwirkung von Sedimentationskräften auf den Kolben (3) keine Flüssigkeit aus dem Inneren des Zylinders (1) über das dann verschlossene, freie Ende (14) des vollständig oder teilweise transparenten Rohres (5) unbeabsichtigt austreten kann.

Gemäß Figur 3 kann in besonders bevorzugten Ausführungsformen an der Innenseite des Deckels (30) zusätzlich eine Einrichtung (44) für den deckelzentrierenden Eingriff bei geschlossenem Deckel (30) - zwischen das Außengewinde (34) der Anschlusseinrichtung (7) einerseits und die Innenwand des Behälters (28) andererseits - vorgesehen sein.

In besonders bevorzugten Ausführungsformen der erfindungsgemäßen Vorrichtung kann der insbesondere in Figur 3 dargestellte Deckel (30) der Verschlusskappe (6) in seiner geschlossenen, auf dem Rand (29) der Verschlusskappe (6) aufliegenden Position und/oder in seiner aufgeschwenkten Position reversibel arretierbar sein.

Vorzugsweise kommt in der geschlossenen Position des Deckels (30) die an der Innenseite des Deckels (30) vorgesehene Einrichtung (43) dicht mit dem innerhalb des Behälters (28) der Verschlusskappe (6) angeordneten Konus (32) in Eingriff.

Insbesondere aus Figur 3 geht hervor, dass die Anschlusseinrichtung (38) für eine dichte Verbindung zwischen der Anschlusseinrichtung (7) für eine Spritze (8) einerseits und dem oberen, freien Ende (14) des Rohres (5) andererseits einen den äußeren Umfang des vollständig oder teilweise transparenten Rohres (5) umschließenden Sockel (39) umfassen kann.

Dieser Sockel (39) kann - in der Draufsicht - in seinem Mittenbereich eine sich vertikal erstreckende Aussparung (42) aufweisen und ein Innengewinde (40) tragen, wobei ein hierzu korrespondierendes Außengewinde (41) auf der Außenseite des obenliegenden Endabschnittes (14) des Rohres (5) vorgesehen sein kann.

Die Figur 7 zeigt, dass der Verschluss (45) zum Verschließen der Spitze (46) des Zylinders (1) beispielsweise einen flüssigkeitsdicht mit der konusförmig sich nach unten verjüngenden Spitze (46) des Zylinders (1) in Kontakt bringbaren, zentralen Konus (47) umfassen kann.

Vorzugsweise kann die Außenseite des Ringes (51), in welchem innenseitig der zentrale Konus (47) ausgebildet ist, ein Außengewinde (48) tragen. Das Außengewinde (48) kann mit dem hierzu korrespondierenden Innengewinde (49) eines allseitig die Spitze (46) des Zylinders (1) umfassenden Ringes (50) in Eingriff bringbar sein.

Figur 4 offenbart, dass - anstelle einer Verschlusskappe (6) - an dem oberen, freien Ende (14) des Rohres (5) ein Verschluss-Deckel (53) mittelbar oder unmittelbar anbringbar sein kann.

In der Regel kann der sich über die Außenseite des Rohres (5) horizontal nach außen erstreckende Überstand (54) des Verschlussdeckels (53) - zur Fingerauflage während des aspiratorischen Anhebens des Kolbens (3) - in dem Bereich von 5,0 mm bis 40,0 mm, vorzugsweise in dem Bereich von 7,0 mm bis 35,0 mm, insbesondere in dem Bereich von 9,0 mm bis 30,0 mm liegen.

Gemäß Figur 4 kann an der Unterseite des Verschluss-Deckels (53) ein sich konusartig nach unten verjüngernder Zapfen (55) zum dichten Verschließen des Konus (60) einer Anschlusseinrichtung (59) für eine Spritze (8) vorgesehen sein.

Zusätzlich zu dem Zapfen (55) kann gegebenenfalls eine Wandung (56) an der Unterseite des Verschluss-Deckels (53) vorgesehen sein, welche ringförmig und von dem Zapfen (55) beabstandet, den zentralen Zapfen (55) umschließt.

In der Regel ist der Zapfen (55) - in vertikaler Hinsicht - kürzer als die ihn umschließende ringförmige Wandung (56) ausgebildet.
Dies führt zu dem Vorteil, dass eine Kontamination des kurzen sterilen Zapfens (55) durch Keime selbst bei einer keimbehafteten Berührung der in vertikaler Hinsicht längeren ringförmigen Wandung (56) sicher ausgeschlossen wird, wodurch eine Kontamination des Konus (32) für eine Spitze (33) der Spritze (8) vermieden wird.

In besonders bevorzugten Ausführungsformen kann die an der Unterseite des Verschluss-Deckels (53) angebrachte ringförmige Wandung (56) innenseitig ein Innengewinde (57) für den Eingriff in ein an der Außenseite der Wandung (61) der Einrichtung (59) vorgesehenes Außengewinde (58) tragen. Dieses Außengewinde (58) kann also vorzugsweise außenseitig an der den Konus (60) der Einrichtung (59) ringförmig umfassenden Wandung (61) vorgesehen sein, wobei die Wandung (61) der Einrichtung (59) außenseitig von einer ringförmigen Wandung (62) umschlossen wird.

Die in Figur 4 dargestellte ringförmige Wandung (62) kann mit dem Sockel (39) der Einrichtung (59) oder mit der Unterseite eines Verschluss-Deckels (53) einstückig in Verbindung stehen.

In der Regel kann die Wandung (62) von dem Außengewinde (58) der den zentralen Konus (60) tragenden Wand (61) zumindest etwas beabstandet sein.

Gemäß Figur 4 kann in besonders bevorzugten Ausführungsformen ferner die Einrichtung (59) zur dichten Anbringung des Verschluss-Deckels (53) in ihrem nach unten weisenden Bereich einen den äußeren Umfang des freien Endes (14) des vollständig oder teilweise transparenten Rohres (5) umschließenden Sockel (39) für eine dichte Verbindung zwischen der Anschlusseinrichtung (59) für eine Spritze (8) einerseits und dem nach oben weisenden, freien Ende (14) des Rohres (5) andererseits, aufweisen.

Dieser Sockel (39) kann - in der Draufsicht - beispielsweise ein Innengewinde (40) tragen, wobei ein hierzu korrespondierendes Außengewinde (41) auf der Außenseite des Endabschnittes (14) des Rohres (5) vorgesehen sein kann.

In besonders bevorzugten Ausführungsformen der erfindungsgemäßen Vorrichtung kann der Abstand zwischen dem Innendurchmesser (13) des Rohres (5) und der Außenseite des transparenten Rohres (5) - bei vertikal ausgerichtetem Rohr (5) - in der Nähe des Kolbenträgers (23) einerseits und des wannen- oder becherförmigen Behälters (28) der Verschlusskappe (6) andererseits linsenförmig kleiner sein als in dem dazwischenliegenden Mittenbereich des Rohres (5).

Diese gegebenenfalls ausgebildete linsenförmige Verdickung der Wand des transparenten Rohres (5) führt zu dem Vorteil, dass der Phasengrenzbereich zwischen zwei voneinander zu trennenden Phasen optisch linsenförmig vergrößernd aufgespreizt und in visueller Hinsicht besonders deutlich erkennbar dargestellt wird. Hierdurch wird eine besonders vollständige und - in Bezug auf die noch in dem Kolben (3) verbleibende, dichtere Phase - besonders sichere vermischungsfreie Entnahme der darüber ausgebildeten Phase geringerer Dichte ermöglicht.

Alternativ oder zusätzlich hierzu kann diese linsenartige Verdickung der Wand des Rohres (5) in dem zwischen der Mitte des Rohres (5) einerseits und dem wannen- oder becherförmigen Behälter (28) der Verschlusskappe (6) andererseits gelegenen Bereich vorgesehen sein.

Zusammenfassend ist festzustellen, dass im Rahmen der vorliegenden Erfindung eine Vorrichtung zum Aufnehmen von Flüssigkeiten - insbesondere von Blut - sowie zur exakten Entnahme von durch Einwirkung von Sedimentationskräften erzeugten einzelnen Phasen der aufgenommenen Flüssigkeit, bereitgestellt wird.

Ein besonderer Vorteil der erfindungsgemäßen Vorrichtung besteht darin, dass sie eine besonders einfache, schnelle und exakte Gewinnung einzelner Phasen (z.B. A, B oder C) unterschiedlicher Dichten gestattet.

Die erfindungsgemäße Vorrichtung ist auch deswegen vorteilhaft, weil sie die Gefahr einer Einbringung von Keimen während der Entnahme von einzelnen Phasen sicher ausschließt.

Ein besonderer Vorteil der erfindungsgemäßen Vorrichtung ist darin zu sehen, dass sie die Isolierung und Entnahme selbst sehr dünner, kleinvolumiger Grenzschichten (z.B. B) - unter Ausschluss der Gefahr von Durchmischung mit der darüber (z.B. C) oder darunter (z.B. A) befindlichen Phase - gestattet.

Schließlich ist die erfindungsgemäße Vorrichtung auch deswegen vorteilhaft, weil sie nicht nur die teilweise, sondern sogar die nahezu vollständige Entnahme des Volumens der jeweils interessierenden Phase erlaubt.

## Patentansprüche

1. Vorrichtung zum Aufnehmen von Flüssigkeiten sowie zur exakten Entnahme von einzelnen Phasen der aufgenommenen Flüssigkeit, welche einen Zylinder (1) umfasst, in welchem ein Kolben (3) entlang der Längsachse (2) des Zylinders (1) reversibel hin- und her- bewegbar ist, wobei der Kolben-Boden (11, 65) - in der Draufsicht - in seinem Mittenbereich eine lochförmige Aussparung (4) aufweist, welche - durch den Kolben (3) hindurch - mit einem entlang der Längsachse (2) des Zylinders (1) ausgerichteten, sich stangenseitig erstreckenden und vollständig oder teilweise transparenten Rohr (5) mittelbar oder unmittelbar in dichter Verbindung steht oder mittelbar oder unmittelbar in dichte Verbindung bringbar ist, wobei der Innendurchmesser (13) des Lumens (52) des Rohres (5) in dem Bereich von 0,5 mm bis 10,0 mm liegt, die Länge des Rohres (5) und dessen Lumens (52) in dem Bereich von 5,0 cm bis 15,0 cm liegt und wobei an dem - bei vertikaler Ausrichtung des Zylinders (1) - in vertikaler Hinsicht nach oben ragenden, freien Ende (14) des Rohres (5) eine reversibel verschließbare Verschlusskappe (6) vorgesehen ist, wobei in der Verschlusskappe (6) eine schraubgewindeförmige und/oder konusförmige Anschlusseinrichtung (7) für eine Injektionsspritze (8) vorgesehen ist und wobei ein sich über die Außenseite des Rohres (5) horizontal nach außen erstreckender Überstand (54) der Verschlusskappe (6) - zur Fingerauflage während des aspiratorischen Anhebens des Kolbens (3) dient - und in dem Bereich von 5,0 mm bis 40,0 mm liegt und wobei in dem Bereich der Mitte des Bodens (9) des Zylinders (1) außenseitig eine schraubengewindeförmige und/oder konusförmige Anschlusseinrichtung (10) für eine Kanüle oder für einen aufschraubbaren oder aufsteckbaren Standfuß (12) vorgesehen sind und wobei in dem dem Boden (9) gegenüberliegenden, oberen Bereich des freien Endes des Zylinders (1) außen umlaufend oder gegenüberliegend Vorsprünge (15) zur Fingerauflage vorgesehen sind, **dadurch gekennzeichnet, dass** die Verschlusskappe (6) einen im Querschnitt U-förmigen oder einen im Querschnitt einem um 90° zur Seite geneigten Buchstaben C entsprechenden Behälter (28) umfasst, wobei mittelbar oder unmittelbar an einem Bereich des freien Randes (29) dieses Behälters (28) ein Deckel (30) für das mittelbare oder unmittelbare Verschließen der obenliegenden Öffnung des Behälters (28) und für das mittelbare oder unmittelbare Verschließen des - innerhalb des Behälters (28) vorgesehenen - Konus (32) einer Anschlusseinrichtung (7) für eine dichte Aufnahme der Spitze (33) einer Spritze (8), über ein Gelenk (31) reversibel schwenkbar angebracht ist und wobei in dem untenliegenden Bodenbereich des Behälters (28) eine vertikal nach oben ausgerichtete Anschlusseinrichtung (7) für eine Spritze (8) vorgesehen ist, welche einen Konus (32) zur Zentrierung und Arretierung der Spitze (33) einer Spritze (8) sowie ein Außengewinde (34) für den Eingriff eines die Spitze (33) ringförmig umfassenden, an einem ringförmigen Vorsprung (35) angebrachten Innengewindes (36), aufweist, wobei der Behälter (28) und die Anschlusseinrichtung (7) für eine Spritze (8) einstückig miteinander ausgebildet sind oder über ein Gewinde (37), eine Einrast-, Schnapp-, Steck-, Dreh- oder einer Bajonette- Verbindung miteinander in permanente oder reversible Verbindung bringbar sind, wobei die Anschlusseinrichtung (7) für eine Spritze (8) stromabwärts und in vertikal nach unten ausgerichteter Richtung mittelbar oder unmittelbar mit einer Anschlusseinrichtung (38) für das freie, obere Ende des Rohres (5) in Verbindung steht, wobei an der Innenseite des Deckels (30) eine ein- oder mehrteilige Einrichtung (43) zum mittelbaren oder unmittelbaren, dichten Verschließen des Konus (32) der Anschlusseinrichtung (7) vorgesehen ist und wobei an der Innenseite des Deckels (30) eine Einrichtung (44) für den deckelzentrierenden Eingriff bei geschlossenem Deckel (30) zwischen das Außengewinde (34) der Anschlusseinrichtung (7) einerseits und die Innenwand des Behälters (28) andererseits, vorgesehen ist.

2. Vorrichtung zum Aufnehmen von Flüssigkeiten sowie zur exakten Entnahme von einzelnen Phasen der aufgenommenen Flüssigkeit nach Anspruch 1, **dadurch gekennzeichnet, dass** das vollständig oder teilweise transparente Rohr (5) mit seinem Lumen (52) an der Stangenseite (17) des Kolbens (3) mittels einer permanent fixierenden oder reversibel lösbaren Einrast-, Schraub-, Schnapp-, Steck-, Dreh- oder einer Bajonette-Verbindung anbringbar ist.

3. Vorrichtung zum Aufnehmen von Flüssigkeiten sowie zur exakten Entnahme von einzelnen Phasen der aufgenommenen Flüssigkeit nach einem der Ansprüche 1 oder 2, **dadurch gekennzeichnet, dass** das vollständig oder teilweise transparente Rohr (5) mit seinem Lumen (52) an der Stangenseite (17) des Kolbens (3) mittels einer konusförmigen und/oder schraubgewindeförmigen, in den Kolben (3) integrierten Anschlusseinrichtung (16) reversibel anbringbar ist, wobei diese Anschlusseinrichtung (16) einen zentralen Konus (18) für die Ausrichtung und Arretierung einer Spitze (19) des Rohres (5) und außenseitig an einem ringförmig den Konus (18) wandartig umschließenden Element (22) ein Außengewinde (20) für das Aufschrauben des Innengewindes (21) eines ringförmig die Spitze (19) des Rohres (5) umschließenden Elementes (22), umfasst.

4. Vorrichtung zum Aufnehmen von Flüssigkeiten sowie zur exakten Entnahme von einzelnen Phasen der aufgenommenen Flüssigkeit nach Anspruch 1, **dadurch gekennzeichnet, dass** das vollständig oder teilweise transparente Rohr (5) mit seinem Lumen (52) einstückig mit der Stangenseite (17) des Kolbenträgers (23) des Kolbens (3) ausgebildet ist.

5. Vorrichtung zum Aufnehmen von Flüssigkeiten sowie zur exakten Entnahme von einzelnen Phasen der aufgenommenen Flüssigkeit nach Anspruch 4, **dadurch gekennzeichnet, dass** das vollständig oder teilweise transparente Rohr (5) mit seinem Lumen (52) einstückig mit der Stangenseite (17) des Kolbenträgers (23) des Kolbens (3) ausgebildet ist, wobei zwischen den Außenseiten des Rohres (5) und dem stangenseitigen Boden (25) des Kolbens (3) ein, zwei, drei, vier, fünf, sechs oder mehrere Versteifungselemente (24) vorgesehen sind, welche sich in vertikaler Hinsicht über die gesamte vertikale Höhe des Rohres (5) - oder über einen Teil hiervon - erstrecken und welche sich, in horizontaler Hinsicht, über die gesamte Breite des stangenseitigen Bodens (25) des Kolbenträgers (23) - oder über einen Teil hiervon - zwischen der Außenseite des zentralen Rohres (5) und dem außenliegenden, kreisförmigen Rand des Bodens (25) des Kolbens (3) erstrecken, um durch eine Abstützung an den Innenwänden des Zylinders (1) ein seitliches Kippen des Kolbens (3) während der Einwirkung von Sedimentationskräften zu verhindern.

6. Vorrichtung zum Aufnehmen von Flüssigkeiten sowie zur exakten Entnahme von einzelnen Phasen der aufgenommenen Flüssigkeit nach einem der vorhergehenden Ansprüche 1 bis 5, **dadurch gekennzeichnet, dass** der Kolbenträger (23) - zur Abdichtung des Kolbens (3) gegenüber den Innenwänden des Zylinders (1) - eine kappenförmige Dichtung (26) mit einem, zwei, drei, vier, fünf oder sechs Dichtungswülsten (27) trägt, welche aus einem elastischen Material gefertigt ist und dass zwischen der trichterförmigen, bodenseitigen Wandung (63) des Bodens (65) der kappenförmigen Dichtung (26) des Kolben-Trägers (23) einerseits und einer zwischen den unteren ringförmig umlaufenden, gegenüberliegenden Dichtungswülsten (27) der kappenförmigen Dichtung (26) gedachten Horizontalen (64) andererseits, ein Winkel α eingeschlossen ist, welcher in dem Bereich von + 35° bis - 35° liegt und wobei in dem Boden (65) der kappenförmigen Dichtung (26) eine zentrale lochförmige Aussparung vorgesehen ist, welche mit der lochförmigen Aussparung (4) des Kolbens (3) durchgehend fluchtet.

7. Vorrichtung zum Aufnehmen von Flüssigkeiten sowie zur exakten Entnahme von einzelnen Phasen der aufgenommenen Flüssigkeit nach Anspruch 1, **dadurch gekennzeichnet, dass** der Deckel (30) der Verschlusskappe (6) in seiner geschlossenen, auf dem Rand (29) der Verschlusskappe (6) aufliegenden Position und/oder in seiner aufgeschwenkten Position arretierbar ist, wobei in seiner geschlossenen Position die an der Innenseite des Deckels (30) vorgesehene Einrichtung (43) dicht mit dem innerhalb des Behälters (28) angeordneten Konus (32) in Eingriff kommt.

8. Vorrichtung zum Aufnehmen von Flüssigkeiten sowie zur exakten Entnahme von einzelnen Phasen der aufgenommenen Flüssigkeit nach einem der vorhergehenden Ansprüche 1 bis 7, **dadurch gekennzeichnet, dass** die Anschlusseinrichtung (38) für eine dichte Verbindung zwischen der Anschlusseinrichtung (7) für eine Spritze (8) einerseits und dem oberen, freien Ende (14) des Rohres (5) andererseits einen den äußeren Umfang des vollständig oder teilweise transparenten Rohres (5) umschließenden Sockel (39) umfasst, welcher - in der Draufsicht - in seinem Mittenbereich eine sich vertikal erstreckende Aussparung (42) aufweist und ein Innengewinde (40) trägt, wobei ein hierzu korrespondierendes Außengewinde (41) auf der Außenseite des obenliegenden Endabschnittes (14) des Rohres (5) vorgesehen ist.

9. Vorrichtung zum Aufnehmen von Flüssigkeiten sowie zur exakten Entnahme von einzelnen Phasen der aufgenommenen Flüssigkeit nach einem der vorhergehenden Ansprüche 1 bis 8, **dadurch gekennzeichnet, dass** der Verschluss (45) zum Verschließen der Spitze (46) des Zylinders (1) einen flüssigkeitsdicht mit der konusförmig sich nach unten verjüngenden Spitze (46) des Zylinders (1) in Kontakt bringbaren, zentralen Konus (47) umfasst, wobei die Außenseite des Ringes (51), in welchem innenseitig der zentrale Konus (47) ausgebildet ist, ein Außengewinde (48) trägt, welches mit dem hierzu korrespondierenden Innengewinde (49) eines allseitig die Spitze (46) des Zylinders (1) umfassenden Ringes (50) in Eingriff bringbar ist.

10. Vorrichtung zum Aufnehmen von Flüssigkeiten sowie zur exakten Entnahme von einzelnen Phasen der aufgenommenen Flüssigkeit nach einem der vorhergehenden Ansprüche 1 bis 9, **dadurch gekennzeichnet, dass** - anstelle einer Verschlusskappe (6) - an dem oberen, freien Ende (14) des Rohres (5) ein Verschluss-Deckel (53) mittelbar oder unmittelbar anbringbar ist, wobei der sich über die Außenseite des Rohres (5) horizontal nach außen erstreckende Überstand (54) des Verschlussdeckels (53) - zur Fingerauflage während des aspiratorischen Anhebens des Kolbens (3) - in dem Bereich von 5,0 mm bis 40,0 mm liegt, wobei an der Unterseite des Verschluss-Deckels (53) ein sich konusartig nach unten verjüngernder Zapfen (55) zum dichten Verschließen des Konus (60) einer Anschlusseinrichtung (59) für eine Spritze (8) vorgesehen und wobei an der Unterseite des Verschluss-Deckels (53) eine Wandung (56) vorgesehen ist, welche ringförmig und von dem Zapfen (55) beabstandet, den zentralen Zapfen (55) umschließt, wobei der Zapfen (55) - in vertikaler Hinsicht - kürzer als die ihn umschließende ringförmige Wandung (56) ist und wobei die an der Unterseite des Verschluss-Deckels (53) angebrachte ringförmige Wandung (56) innenseitig ein Innengewinde (57) für den Eingriff in ein an der Außenseite der Wandung (61) der Einrichtung (59) vorgesehenes Außengewinde (58) trägt, das außenseitig an der den Konus (60) der Einrichtung (59) ringförmig umfassenden Wandung (61) vorgesehen ist, wobei die Wandung (61) der Einrichtung (59) außenseitig von einer ringförmigen Wandung (62) umschlossen wird, die mit dem Sockel (39) der Einrichtung (59) oder mit der Unterseite des Deckels (53) einstückig in Verbindung steht, wobei die Wandung (62) von dem Außengewinde (58) der den zentralen Konus (60) tragenden Wand (61) zumindest etwas beabstandet ist und wobei die Einrichtung (59) zur dichten Anbringung des Verschluss-Deckels (53) in ihrem nach unten weisenden Bereich einen den äußeren Umfang des freien Endes (14) des vollständig oder teilweise transparentes Rohres (5) umschließenden Sockel (39) für eine dichte Verbindung zwischen der Anschlusseinrichtung (59) für eine Spritze (8) einerseits und dem nach oben weisenden, freien Ende (14) des Rohres (5) andererseits, aufweist, wobei der Sockel (39) - in der Draufsicht - ein Innengewinde (40) trägt und wobei ein hierzu korrespondierendes Außengewinde (41) auf der Außenseite des Endabschnittes (14) des Rohres (5) vorgesehen ist.

11. Vorrichtung zum Aufnehmen von Flüssigkeiten sowie zur exakten Entnahme von einzelnen Phasen der aufgenommenen Flüssigkeit nach einem der vorhergehenden Ansprüche 1 bis 10, **dadurch gekennzeichnet, dass** der Abstand zwischen dem Innendurchmesser (13) des Rohres (5) und der Außenseite des transparenten Rohres (5) - bei vertikal ausgerichtetem Rohr (5) - in der Nähe des Kolbenträgers (23) einerseits und des wannen- oder becherförmigen Behälters (28) der Verschlusskappe (6) andererseits kleiner ist als in dem dazwischenliegenden Mittenbereich des Rohres (5) und/oder wobei diese Verdickung der Wand des Rohres (5) in dem zwischen der Mitte des Rohres (5) einerseits und dem wannen- oder becherförmigen Behälter (28) der Verschlusskappe (6) andererseits gelegenen Bereich vorgesehen ist.

## Claims

1. An apparatus for receiving fluids and for precise extraction of individual phases of the received fluid, which comprises a cylinder (1) in which a plunger (3) is reversibly movable back and forth along the longitudinal axis (2) of the cylinder (1), wherein the plunger bottom (11, 65) - when viewed from above - has in its middle area a hole-like cut-out (4), which is indirectly or directly connected in a fluid-tight manner or capable of being indirectly or directly connected in a fluid-tight manner - through the plunger (3) - to a completely or partially transparent tube (5) extending on the rod side and aligned along the longitudinal axis (2) of the cylinder (1), wherein the inner diameter (13) of the lumen (52) of the tube (5) is in the range of 0.5 mm to 10.0 mm, the length of the tube (5) and of the lumen (52) thereof is in the range of 5.0 cm to 15.0 cm, and wherein a reversibly closeable end cap (6) is provided on the free end (14) of the tube (5) projecting vertically upwards (with the cylinder (1) aligned vertically), wherein a screw thread-like and/or conical coupler device (7) for a syringe (8) is provided in the end cap (6) and wherein a protrusion (54) of the end cap (6) extending horizontally outwards beyond the outside of the tube (5) serves as a finger support during the aspiratory movement of the plunger (3) and is in the range of 5.0 mm to 40 mm and wherein in the zone of the center of the bottom (9) of the cylinder (1), provision is made of a screw thread-like and/or conical coupler device (10) on the outside for a canula or for a screw-on or push-on pedestal (12) and wherein in the upper region of the free end of the cylinder (1) opposite the bottom (9), outer circumferential or opposing projections (15) are provided for finger support, **characterized in that** the end cap (6) comprises a container (28) with a U-shaped cross section or with a cross section corresponding to a letter C tilted by 90° to the side, wherein a lid (30) for the indirect or direct closure of the upper opening of the container (28) and for the indirect or direct closure of the cone (32) - provided in the container (28) - of a coupler device (7) for sealingly accommodating the tip (33) of a syringe (8) is indirectly or directly reversibly hinge-connected to an area of the free edge (29) of this container by means of a hinge (31) and wherein in the lower bottom zone of the container (28), provision is made of a vertically upwardly aligned coupler device (7) for a syringe (8), which has a cone (32) for centering and locking the tip (33) of a syringe (8) and a male thread (34) for the engagement of a female thread (36) which annularly surrounds the tip (33) and which is arranged on an annular projection (35), wherein the container (28) and the coupler device (7) for a syringe (8) are formed integrally with each other or are reversibly or permanently interconnectable by means of a thread (37), a latch connection, a snap-in connection, a plug-in connection, a rotary connection or a bayonet connection, wherein the coupler device (7) for a syringe (8) is connected downstream and in a vertically downwardly aligned direction indirectly or directly to a coupler device (38) for the free, upper end of the tube (5), wherein a one-piece or multi-piece device (43) for indirect or direct fluid-tight closure of the cone (32) of the coupler device (7) is provided on the inside of the lid (30) and wherein a device (44) is provided on the inside of the lid (30) for a lid-centering engagement between the male thread (34) of the coupler device (7) and the inner wall of the container (28) when the lid (30) is closed.

2. The apparatus for receiving fluids and for precise extraction of individual phases of the received fluid according to Claim 1, **characterized in that** the completely or partially transparent tube (5) with its lumen (52) can be attached to the rod side (17) of the plunger (3) by means of a permanently fixing or reversibly releasable latch connection, screw connection, snap-in connection, plug-in connection, rotary connection or bayonet connection.

3. The apparatus for receiving fluids and for precise extraction of individual phases of the received fluid according to either one of Claims 1 or 2, **characterized in that** the completely or partially transparent tube (5) with its lumen (52) can be reversibly attached to the rod side (17) of the plunger (3) by means of a conical and/or screw thread-like coupler device (16) integrated in the plunger (3), wherein said coupler device (16) comprises a central cone (18) for aligning and locking a tip (19) of the tube (5) and further comprises, on the outside on an element (22) surrounding the cone (18) in an annular, wall-like manner, a male thread (20) for screwing on the female thread (21) of an element (22) annularly surrounding the tip (19) of the tube (5).

4. The apparatus for receiving fluids and for precise extraction of individual phases of the received fluid according to Claim 1, **characterized in that** the completely or partially transparent tube (5) with its lumen (52) is formed integrally with the rod side (17) of the plunger carrier (23) of the plunger (3).

5. The apparatus for receiving fluids and for precise extraction of individual phases of the received fluid according to Claim 4, **characterized in that** the completely or partially transparent tube (5) with its lumen (52) is formed integrally with the rod side (17) of the plunger carrier (23) of the plunger (3), wherein between the outsides of the tube (5) and the rod-side bottom (25) of the plunger (3), provision is made of one, two, three, four, five, six or more reinforcing elements (24), which extend in a vertical respect over the entire vertical height of the tube (5) - or over a portion thereof - and which extend in a horizontal respect over the entire width of the rod-side bottom (25) of the plunger carrier (23) - or over a portion thereof - between the outside of the central tube (5) and the outer circular edge of the bottom (25) of the plunger (3) such that the plunger (3), by being supported on the inner walls of the cylinder (1), is prevented from tilting sideways during the application of sedimentation forces.

6. The apparatus for receiving fluids and for precise extraction of individual phases of the received fluid according to any one of preceding Claims 1 to 5, **characterized in that**, for sealing the plunger (3) with respect to the inner walls of the cylinder (1), the plunger carrier (23) bears a cap-like seal (26) having one, two, three, four, five or six sealing beads (27), which is made of an elastic material, and that an angle α ranging from + 35° to - 35° is defined between the funnel-shaped, bottom-side partition (63) of the bottom (65) of the cap-like seal (26) of the plunger carrier (23) and an imaginary horizontal line (64) between the lower annularly surrounding, opposing sealing beads (27) of the cap-like seal (26), and wherein a central hole-like cut-out is provided in the bottom (65) of the cap-like seal (26), which aligns continuously with the hole-like cut-out (4) of the plunger (3).

7. The apparatus for receiving fluids and for precise extraction of individual phases of the received fluid according to Claim 1, **characterized in that** the lid (30) of the end cap (6) is lockable in its closed position in abutment on the edge (29) of the end cap (6) and/or in its outwardly-swiveled position, wherein in its closed position, the device (43) provided on the inside of the lid (30) comes in sealing engagement with the cone (32) arranged inside the container (28).

8. The apparatus for receiving fluids and for precise extraction of individual phases of the received fluid according to any one of preceding Claims 1 to 7, **characterized in that** the coupler device (38) for a fluid-tight connection between the coupler device (7) for a syringe (8) and the upper free end (14) of the tube (5) comprises a base (39) enclosing the outer periphery of the completely or partially transparent tube (5), which - when viewed from above - has a vertically extending cut-out (42) and bears a female thread (40) in its middle area, wherein a male thread (41) corresponding thereto is provided on the outside of the upper end section (14) of the tube (5).

9. The apparatus for receiving fluids and for precise extraction of individual phases of the received fluid according to any one of preceding Claims 1 to 8, **characterized in that** the seal (45) for sealing the tip (46) of the cylinder (1) comprises a central cone (47) capable of being brought into fluid-tight contact with the conically downwardly tapering tip (46) of the cylinder (1), wherein the outside of the ring (51), inside of which the central cone (47) is formed, bears a male thread (48) which can be brought in engagement with the corresponding female thread (49) of a ring (50) surrounding the tip (46) of the cylinder (1).

10. The apparatus for receiving fluids and for precise extraction of individual phases of the received fluid according to any one of preceding Claims 1 to 9, **characterized in that** - in lieu of an end cap (6) - a lid (53) can be attached indirectly or directly to the upper, free end (14) of the tube (5), wherein the protrusion (54) of the lid (53) extending horizontally outwards beyond the outside of the tube (5) - for finger support during the aspiratory movement of the plunger (3) - is in the range of 5.0 mm to 40.0 mm, wherein a conically downwardly tapering plug (55) for sealing the cone (60) of a coupler device (59) for a syringe (8) is provided on the underside of the lid (53) and wherein a partition (56), which surrounds the central plug (55) annularly and at a distance from said plug (55), is provided on the underside of the lid (53), wherein the plug (55) - in a vertical respect - is shorter than the annular partition (56) surrounding the same and wherein the annular partition (56) arranged on the underside of the lid (53) bears on its inside a female thread (57) for engagement with a male thread (58) provided on the outside of the partition (61) of the device (59), said male thread being provided on the outside on the partition (61) annularly surrounding the cone (60) of the device (59), wherein the partition (61) of the device (59) is enclosed on the outside by an annular partition (62) which is integrally connected to the base (39) of the device (59) or to the underside of the lid (53), wherein the partition (62) is at least somewhat distanced from the male thread (58) of the wall (61) bearing the central cone (60) and wherein the device (59) for attaching the lid (53) in a fluid-tight manner has, in its downwardly directed area, a base (39) surrounding the outer periphery of the free end (14) of the completely or partially transparent tube (5) for a fluid-tight connection between the coupler device (59) for a syringe (8) and the upwardly directed free end (14) of the tube (5), wherein the base (39) - when viewed from above - bears a female thread (40) and wherein a male thread (41) corresponding thereto is provided on the outside of the end section (14) of the tube (5).

11. The apparatus for receiving fluids and for precise extraction of individual phases of the received fluid according to any one of preceding Claims 1 to 10, **characterized in that** the distance between the inner diameter (13) of the tube (5) and the outside of the transparent tube (5) - with the tube (5) aligned vertically - is smaller in the vicinity of the plunger carrier (23) and of the tub-shaped or cup-shaped container (28) of the end cap (6) than in the interposed middle area of the tube (5) and/or wherein this thickening of the wall of the tube (5) is provided in the area disposed between the middle of the tube (5) and the tub-shaped or cup-shaped container (28) of the end cap (6).

## Revendications

1. Dispositif destiné à recevoir des liquides ainsi qu'au prélèvement exact de phases individuelles du liquide reçu, lequel comprend un cylindre (1) dans lequel un piston (3) est mobile en mouvement de va-et-vient de façon réversible le long de l'axe longitudinal (2) du cylindre (1), sachant que le fond de piston (11, 65), en vue de dessus, comporte dans sa zone centrale un évidement (4) en forme de trou, lequel se trouve, à travers le piston (3), directement ou indirectement en liaison hermétique, ou peut être mis directement ou indirectement en liaison hermétique, avec un tube (5) complètement ou partiellement transparent orienté le long de l'axe longitudinal (2) du cylindre (1) s'étendant du côté tige, sachant que le diamètre intérieur (13) de la lumière (52) du tube (5) se situe dans la plage de 0,5 mm à 10,0 mm, la longueur du tube (5) et la lumière de celui-ci (52) se situent dans la plage de 5,0 cm à 15,0 cm et sachant qu'en orientation verticale du cylindre (1), en vue verticale, un bouchon de fermeture (6) fermable de façon réversible est prévu à l'extrémité (14) libre dressée vers le haut du tuyau (5), sachant que dans le bouchon de fermeture (6) est prévu un dispositif de raccord (7) en forme de filetage et/ou de forme conique pour une seringue d'injection (8) et sachant qu'une saillie (54) du bouchon de fermeture (6) s'étendant sur la face extérieure du tube (5) horizontalement vers l'extérieur, sert d'appui des doigts pendant le relevage d'aspiration du piston (3) et se situe dans la plage de 5,0 mm à 40,0 mm et sachant que dans la zone du milieu du fond (9) du cylindre (1) est prévu en extérieur un dispositif de raccord (10) en forme de filetage et/ou de forme conique pour une canule ou pour un pied (12) vissable ou emboîtable et sachant que dans la zone supérieure opposée au fond (9) de l'extrémité libre du cylindre (1) sont prévues des saillies (15), en périphérie ou s'opposant extérieurement pour l'appui des doigts, **caractérisé en ce que** le bouchon de fermeture (6) comprend un réservoir (28) en forme de U dans la section ou correspondant en section à une lettre C inclinée à 90° sur le côté, sachant que directement ou indirectement sur une zone du bord libre (29) de ce réservoir (28) est disposé de façon pivotable réversible sur une articulation (31) un couvercle (30) pour la fermeture directe ou indirecte de l'ouverture supérieure du réservoir (28) et pour la fermeture directe ou indirecte du cône (32), prévu à l'intérieur du réservoir (28) d'un dispositif de raccord (7) pour une réception hermétique de la pointe (33) d'une seringue (8) et sachant que dans la zone de fond située en bas du réservoir (28) est prévu un dispositif de raccord (7) orienté verticalement vers le haut pour une seringue (8), lequel comporte un cône (32) pour le centrage et le blocage de la pointe (33) d'une seringue (8) ainsi qu'un filetage extérieur (34) pour l'engagement d'un filetage intérieur (36) comprenant en forme annuaire la pointe (33), placé sur une saillie (35) en forme annulaire, sachant que le réservoir (28) et le dispositif de raccord (7) sont constitués l'une avec l'autre en une seule pièce pour une seringue (8) ou un raccord encliquetable, un raccord à déclic, un raccord emboîtable, un raccord pivotant ou un raccord à baïonnette peuvent être placés en liaison permanente ou réversible entre eux par le biais d'un filetage (37), sachant que le dispositif de raccord (7) pour une seringue (8) est en liaison en aval et en direction orientée verticalement vers le bas, directement ou indirectement avec un dispositif de raccord (38) pour l'extrémité libre supérieure du tube (5), sachant que sur le côté intérieur du couvercle (30) est prévu un dispositif (43) en une ou plusieurs parties pour la fermeture directe ou indirecte hermétique du cône (32) du dispositif de raccord (7), et sachant que sur le côté intérieur du couvercle (30) est prévu un dispositif (44) pour l'engagement centrant le couvercle lorsque le couvercle est fermé (30) entre le filetage extérieur (34) du dispositif de raccord (7) d'une part et la paroi intérieure du réservoir (28) d'autre part.

2. Dispositif destiné à recevoir des liquides ainsi qu'au prélèvement exact de phases individuelles du liquide reçu selon la revendication 1, **caractérisé en ce que** le tube complètement ou partiellement transparent (5) avec sa lumière (52) peut être placé sur le côté de tige (17) du piston (3) au moyen d'un raccord encliquetable, un raccord à déclic, un raccord à vis, un raccord emboîtable, un raccord pivotant ou un raccord à baïonnette à fixation permanente ou amovible de façon réversible.

3. Dispositif destiné à recevoir des liquides ainsi qu'au prélèvement exact de phases individuelles du liquide reçu selon l'une quelconque des revendications précédentes 1 ou 2, **caractérisé en ce que** le tube complètement ou partiellement transparent (5) avec sa lumière (52) peut être placé de façon réversible sur le côté de tige (17) du piston (3) au moyen d'un dispositif de raccord (16) de forme conique et/ou en forme de filetage, intégré dans le piston (3), sachant que ce dispositif de raccord (16) comprend un cône central (18) pour l'orientation et le blocage d'une pointe (19) du tube (5) et extérieurement sur un élément (22) entourant comme une paroi dans une forme annulaire le cône (18), un filetage extérieur (20) pour le vissage du filetage intérieur (21) d'un élément (22) entourant de forme annulaire la pointe (19) du tube (5).

4. Dispositif destiné à recevoir des liquides ainsi qu'au prélèvement exact de phases individuelles du liquide reçu selon la revendication 1, **caractérisé en ce que** le tube complètement ou partiellement transparent (5) avec sa lumière (52) est constitué en une seule pièce avec le côté de tige (17) du support de piston (23) du piston (3).

5. Dispositif destiné à recevoir des liquides ainsi qu'au prélèvement exact de phases individuelles du liquide reçu selon la revendication 4, **caractérisé en ce que** le tube complètement ou partiellement transparent (5) avec sa lumière (52) est constitué en une seule pièce avec le côté tige (17) du support de piston (23) du piston (3), sachant qu'entre les côtés extérieurs du tube (5) et le fond côté tige (25) du piston (3) sont prévus un, deux, trois, quatre, cinq, six ou plusieurs éléments de renfort (24), lesquels s'étendent en vue verticale sur toute la hauteur verticale du tube (5), ou sur une partie de celui-ci, et lesquels s'étendent, en vue horizontale, sur toute la largeur du fond côté tige (25) du support de piston (23), ou sur une partie de celui-ci, entre le côté extérieur du tube central (5) et le bord extérieur, circulaire du fond (25) du piston (3) pour éviter par un soutien sur les parois intérieures du cylindre (1), un basculement latéral du piston (3) pendant l'action des forces de sédimentation.

6. Dispositif destiné à recevoir des liquides ainsi qu'au prélèvement exact de phases individuelles du liquide reçu selon l'une quelconque des revendications précédentes 1 à 5, **caractérisé en ce que** le support de piston (23) porte, pour étanchéifier le piston (3) vis-à-vis des parois intérieures du cylindre (1), un joint d'étanchéité (26) en forme de bouchon avec un, deux, trois, quatre, cinq ou six bourrelets d'étanchéité (27), lequel est fabriqué dans un matériau élastique et **en ce qu'**entre la paroi (63) du fond (65) en forme d'entonnoir, côté fond du joint d'étanchéité en forme de bouchon (26) du support de piston (23) d'une part et une ligne horizontale (64) imaginée entre les bourrelets d'étanchéité (27) inférieurs périphériques de forme annulaire, opposés du joint d'étanchéité (26) en forme de bouchon d'autre part, est inscrit un angle α, lequel se situe dans la plage de +35° à - 35° et sachant que dans le fond (65) du joint d'étanchéité en forme de bouchon (26) est prévu un évidement central en forme de trou, lequel vient affleurer en continu l'évidement (4) en forme de trou du piston (3).

7. Dispositif destiné à recevoir des liquides ainsi qu'au prélèvement exact de phases individuelles du liquide reçu selon la revendication 1, **caractérisé en ce que** le couvercle (30) du bouchon de fermeture (6) peut être bloqué dans sa position fermée, reposant sur le bord (29) du bouchon de fermeture (6) et/ou dans sa position ouverte par pivotement, sachant que dans sa position fermée, le dispositif (43) prévu sur le côté intérieur du couvercle (30) vient en prise de façon hermétique avec le cône (32) disposé à l'intérieur du réservoir (28).

8. Dispositif destiné à recevoir des liquides ainsi qu'au prélèvement exact de phases individuelles du liquide reçu selon l'une quelconque des revendications précédentes 1 à 7, **caractérisé en ce que** le dispositif de raccord (38) comprend pour un raccordement hermétique entre le dispositif de raccord (7) pour une seringue (8) d'une part et l'extrémité supérieure, libre (14) du tube (5) d'autre part un socle (39) entourant la périphérie extérieure du tube (5) complètement ou partiellement transparent, lequel, en vue de dessus, comporte dans sa zone centrale un évidement (42) s'étendant verticalement et porte un filetage intérieur (40), sachant qu'un filetage extérieur (41) correspondant à cet effet est prévu sur le côté extérieur de la section finale supérieure (14) du tube (5).

9. Dispositif destiné à recevoir des liquides ainsi qu'au prélèvement exact de phases individuelles du liquide reçu selon l'une quelconque des revendications précédentes 1 à 8, **caractérisé en ce que** la fermeture (45) destinée à fermer la pointe (46) du cylindre (1) comprend un cône (47) central pouvant être mis en contact de façon hermétique aux liquides avec la pointe (46) du cylindre (1) se réduisant en forme de cône vers le bas, sachant que le côté extérieur de la bague (51), dans laquelle est constitué intérieurement le cône central (47), porte un filetage extérieur (48), lequel peut être mis en engagement avec le filetage intérieur (49) correspondant à cet effet d'une bague (50) entourant de tous côtés la pointe (46) du cylindre (1).

10. Dispositif destiné à recevoir des liquides ainsi qu'au prélèvement exact de phases individuelles du liquide reçu selon l'une quelconque des revendications précédentes 1 à 9, **caractérisé en ce qu'**à la place d'un bouchon de fermeture (6), un couvercle de fermeture (53) peut être directement ou indirectement placé sur l'extrémité supérieure libre (14) du tube (5), sachant que la saillie (54) du couvercle de fermeture (53), s'étendant sur le côté extérieur du tube (5) horizontalement vers l'extérieur, destinée à l'appui des doigts pendant le relevage d'aspiration du piston (3), se situe dans la plage de 5,0 mm à 40,0 mm, sachant que sur le côté inférieur du couvercle de fermeture (53) est prévu un pivot (55) de type conique se réduisant vers le bas pour fermer hermétiquement le cône (60) d'un dispositif de raccord (59) pour une seringue (8), sachant que sur le côté inférieur du couvercle de fermeture (53) est prévue une paroi (56), laquelle entoure le pivot central (55) de façon annulaire et à distance du pivot (55), sachant que le pivot (55), en vue verticale, est plus petit que la paroi (56) l'entourant en forme annulaire et sachant que la paroi (56) de forme annulaire placée sur le côté inférieur du couvercle de fermeture (53) porte à l'intérieur un filetage intérieur (57) pour l'engagement dans un filetage extérieur (58) prévu sur le côté extérieur de la paroi (61) du dispositif (59), qui est prévu extérieurement sur la paroi (61) englobant de forme annulaire le cône (60) du dispositif (59), sachant que la paroi (61) du dispositif (59) est entourée extérieurement par une paroi (62) de forme annulaire , qui est en liaison en une seule pièce avec le socle (39) du dispositif (59) ou avec le côté inférieur du couvercle (53), sachant que la paroi (62) est au moins un peu à distance du filetage extérieur (58) de la paroi (61) portant le cône central (60) et sachant que le dispositif (59) comporte, pour un montage hermétique du couvercle de fermeture (53) dans son secteur tourné vers le bas, un socle (39) entourant la périphérie extérieure de l'extrémité libre (14) du tube complètement ou partiellement transparent (5) pour une liaison hermétique entre le dispositif de raccord (59) pour une seringue (8) d'une part et l'extrémité libre tournée vers le haut (14) du tube (5) d'autre part, sachant que le socle (39), en vue de dessus, porte un filetage intérieur (40) et sachant qu'un filetage extérieur (41) correspondant à cet effet est prévu sur le côté extérieur de la section finale (14) du tube (5) .

11. Dispositif destiné à recevoir des liquides ainsi qu'au prélèvement exact de phases individuelles du liquide reçu selon l'une quelconque des revendications précédentes 1 à 10, **caractérisé en ce que** l'intervalle entre le diamètre intérieur (13) du tube (5) et le côté extérieur du tube transparent (5) , avec le tube (5) orienté verticalement, est, à proximité du support de piston (23) d'une part et du réservoir (28) en forme de cuvette ou de godet du bouchon de fermeture (6) d'autre part, plus petit que dans la zone centrale intermédiaire du tube (5) et/ou sachant que cet épaississement de la paroi du tube (5) est prévu dans la zone située entre le centre du tube (5) d'une part et le réservoir (28) en forme de cuvette ou de godet du bouchon de fermeture (6) d'autre part.
